# EUROPÄISCHE PATENTSCHRIFT

(11) **EP 0 400 382 B1**
(45) Veröffentlichungstag und Bekanntmachung des Hinweises auf die Patenterteilung: **13.03.1996**
(21) Anmeldenummer: 90108994.6
(22) Anmeldetag: 12.05.1990
(51) Int. Cl.: C07D 251/50

(54) **Verfahren zur Herstellung von substituierten 2,4-Diamino-6-fluor-s-triazinen**
Process for the preparation of substituted 2,4-diamino-6-fluoro-s-triazines
Procédé pour la préparation de 2,4-diamino-6-fluoro-s-triazines substituées

(30) Priorität: 25.05.1989 DE 3917046
(43) Veröffentlichungstag der Anmeldung: 05.12.1990
(73) Patentinhaber: BAYER AG, D-51368 Leverkusen (DE)
(72) Erfinder: Herd, Karl Josef, Dr., D-5068 Odenthal (DE); Frosch, Hans-Georg, Dr., D-5000 Koeln 91 (DE); Henk, Hermann, Dr., D-5000 Koeln 80 (DE); Müllers, Wolfgang, Dr., D-5060 Bergisch-Gladbach 1 (DE); Stöhr, Frank-Michael, Dr., D-5068 Odenthal (DE)

(56) Entgegenhaltungen:
- EP-A- 0 172 790
- EP-A- 0 227 983
- DE-A- 2 557 141
- DE-A- 2 747 011
- DE-A- 2 903 594

## Beschreibung

Gegenstand der vorliegenden Erfindung ist ein Kondensationsverfahren zur Herstellung von substituierten 2,4-Diamino-6-fluortriazinen der Formel 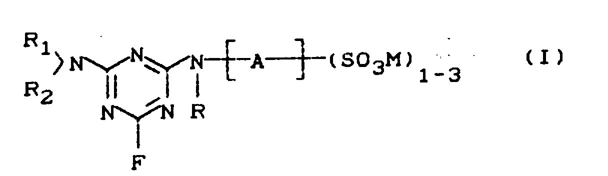 dadurch gekennzeichnet, daß man Ammoniumsalze der Formel 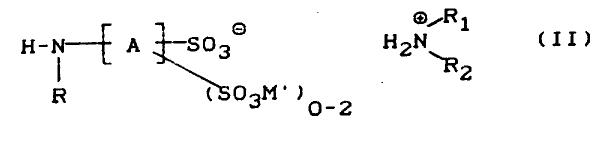 worin
- A =: aromatisch-carbocyclischer oder aromatischheterocyclischer Rest
- M =: Alkalimetallkation
- R =: H oder C₁-C₄-Alkyl
- R₁,R₂=: unabhängig voneinander Wasserstoff, aliphatischer oder cycloaliphatischer Rest oder zusammen mit dem N-Atom ein 5- oder 6-gliedriger heterocyclischer Rest und
- M' =: M oder H bedeuten,
in wäßrigen Medien bei pH-Werten von etwa 2,5-10,0 kontinuierlich mit 2,4,6-Trifluortriazin (III) im Molverhältnis II zu III von 0,8:1 bis 1,5:1 umsetzt, wobei man das Triazin III und eine wässrige Lösung des Ammoniumsalzes (II) gleichzeitig und kontinuierlich in einen ersten Reaktor leitet, dort eine intensive Durchmischung vornimmt, und die Reaktionsmischung anschließend in einen zweiten Reaktor leitet, in dem nur geringe Rückmischung aber gute radiale Vermischung eintritt, und dort die Erstkondensation bei pH 2,5 bis 6 zu Ende führt und anschließend im 2. Reaktor kontinuierlich oder in einem dritten Reaktor kontinuierlich oder diskontinuierlich bei pH 6 bis 10, insbesondere durch Zudosieren von Basen, z.B. Alkalicarbonat oder Alkalihydrogencarbonat, den Austausch des 2. Fluoratoms durchführt.

Geeignete Kationen M sind insbesondere Na⁺, K⁺, Li⁺.

Geeignete Reste A sind insbesondere solche der Benzol- oder Naphthalinreihe, insbesondere solche die noch OH und/oder NH₂-Gruppen aufweisen.

Die Alkylreste R können gegebenenfalls substituiert sein, beispielsweise durch OH, Cl, COOH, SO₃H, OSO₃H.

Geeignete Reste R₁ und R₂ sind beispielsweise gegebenenfalls substituiertes C₁-C₄-Alkyl (Substituenten: OH, CN, COOH, SO₃H, OSO₃H, OC₁-C₄-Alkyl, N(C₁-C₄-Alkyl)₂, C₁-C₄-Alkylcarbonylamino, Phenyl, Sulfophenyl und SO₂X mit
- X =: CH=CH₂, CH₂CH₂OSO₃H, CH₂CH₂Cl, CH₂CH₂SSO₃H, CH₂CH₂OCOCH₃)
bzw. zusammen mit dem N-Atom: 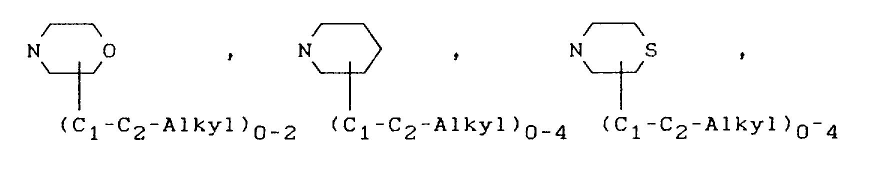 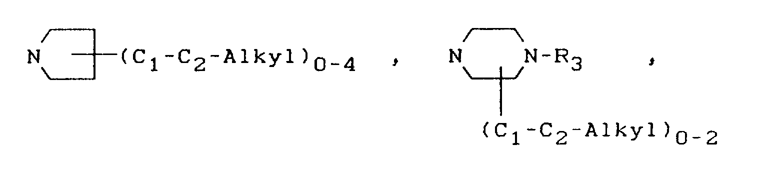 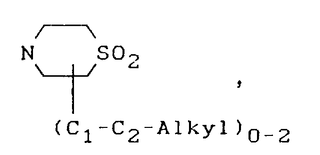 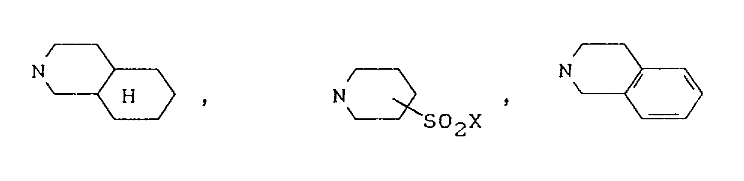 wobei X die oben genannte Bedeutung besitzt und R₃ = H, gegebenenfalls substituiertes C₁-C₄-Alkyl (Substituenten insbesondere OH, OSO₃H, SO₂X).

Im Einzelnen seien folgende Reste R₁ bzw. R₂ genannt:
CH₃, C₂H₅, C₃H₇, C₄H₉ (verzweigt bzw. geradkettig),
CH₂CH₂OH, CH₂CH₂CN, CH₂CO₂H, CH₂CH₂CO₂H, CH₂CH₂SO₃H,
CH₂SO₃H, CH₂CH₂OSO₃H, CH₂CH₂CH₂OH, CH₂CH₂CH₂OSO₃H,
CH₂-CH(OH)-CH₃, CH₂CH₂NHCOCH₃, CH₂CH₂OCH₃,
CH₂CH₂OCH₂CH₃, CH₂CH₂CH₂OC₂H₅, CH₂CH₂N(CH₃)₂,
CH₂CH₂CH₂N(CH₃)₂, Cyclohexyl, Benzyl, Sulfobenzyl, CH₂CH₂-C₆H₅ sowie

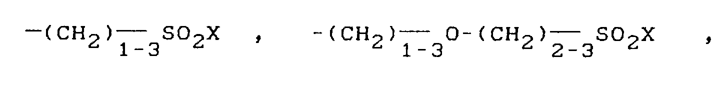

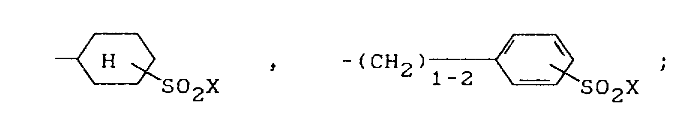

Besonders geeignete Ausgangsprodukte (II) sind solche der Formel 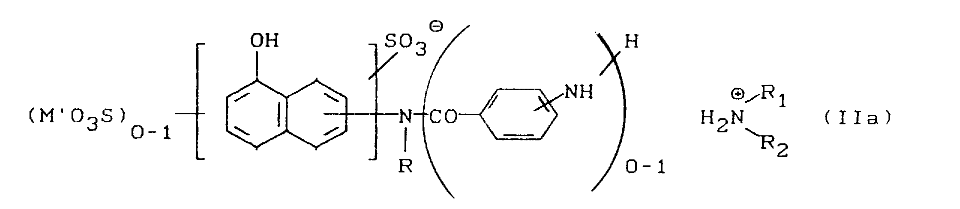 die mit Cyanurfluorid zu Verbindungen der Formel 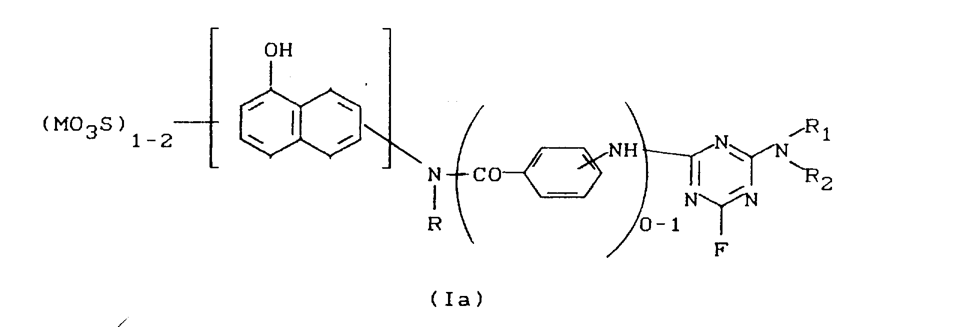 führen.

Die Ammoniumsalze (II) erhält man in bekannter Weise durch Zusatz von 1 Mol Amin HNR₁R₂ (IV) zu der wäßrigen Lösung von 1 Mol Säure

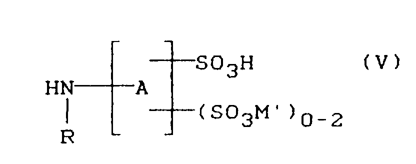

Als Reste 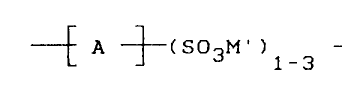 in Form der freien Säuren formuliert - seien im Einzelnen genannt:

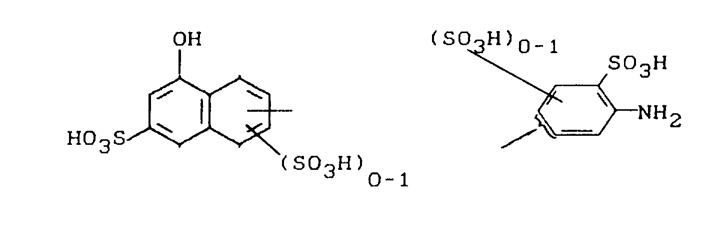

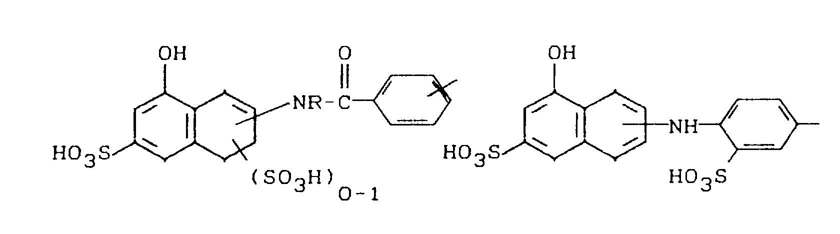

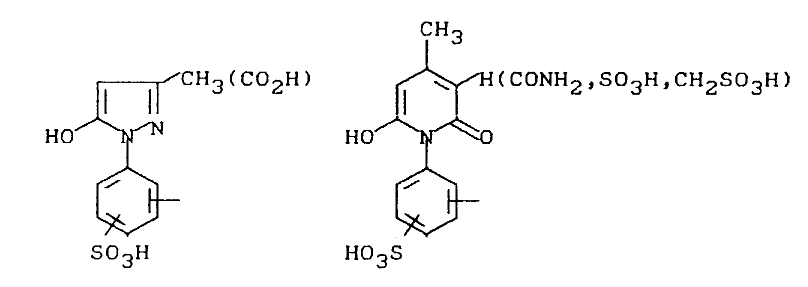

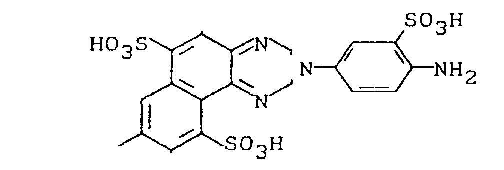

Als Beispiele für die den Ammoniumsalzen (II) zugrundeliegenden Säuren (V) seien genannt:
8-Amino-1-hydroxynaphthalin-3,5-disulfonsäure
8-Amino-1-hydroxynaphthalin-3,6-disulfonsäure
7-Amino-1-hydroxynaphthalin-3,6-disulfonsäure
6-Amino-1-hydroxynaphthalin-3,5-disulfonsäure
6-Amino-1-hydroxynaphthalin-3-sulfonsäure
6-Methylamino-1-hydroxynaphthalin-3-sulfonsäure
7-Amino-1-hydroxynaphthalin-3-sulfonsäure
7-Methylamino-1-hydroxynaphthalin-3-sulfonsäure
5-Amino-1-hydroxynaphthalin-3-sulfonsäure
8-(4-Aminobenzoyl)amino-1-hydroxynaphthalin-3,5-disulfonsäure
8-(2-Aminobenzoyl)amino-1-hydroxynaphthalin-3,5-disulfonsäure
8-(4-Aminobenzoyl)amino-1-hydroxynaphthalin-3,6-disulfonsäure
8-(2-Aminobenzoyl)amino-1-hydroxynaphthalin-3,6-disulfonsäure
8-(3-Aminobenzoyl)amino-1-hydroxynaphthalin-3,6-disulfonsäure
7-(4-Aminobenzoyl)amino-1-hydroxynaphthalin-3-sulfonsäure
6-(2-Aminobenzoyl)amino-1-hydroxynaphthalin-3-sulfonsäure
2,4-Diaminobenzolsulfonsäure
2,5-Diaminobenzolsulfonsäure
2,5-Diaminobenzol-1,3-disulfonsäure
2,5-Diaminobenzol-1,4-disulfonsäure
2,4-Diaminobenzol-1,5-disulfonsäure
6-(4-Amino-2-sulfophenyl)amino-1-hydroxynaphthalin-3-sulfonsäure
7-(4-Amino-2-sulfophenyl)amino-1-hydroxynaphthalin-3-sulfonsäure

Als Beispiele für die den Ammonmiumsalzen (II) zugrundeliegenden chromophoren Amine (V) seien genannt:

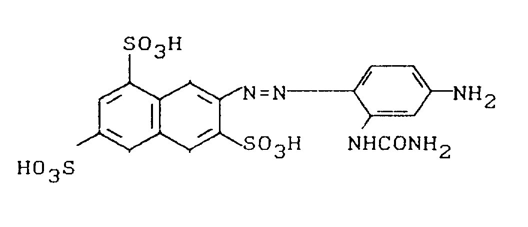

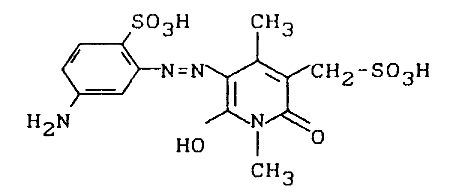

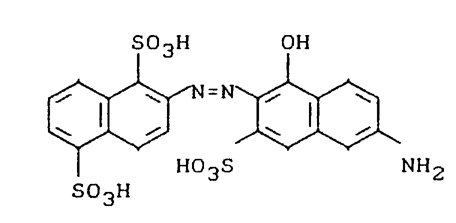

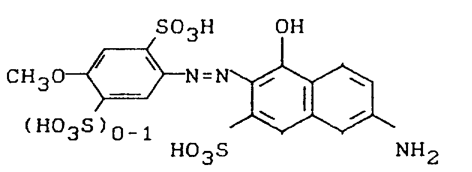

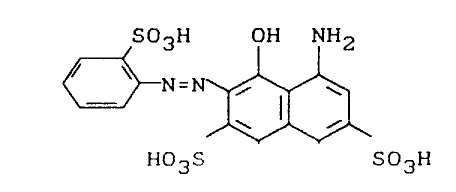

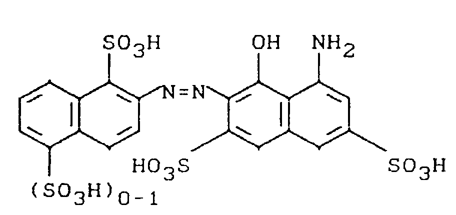

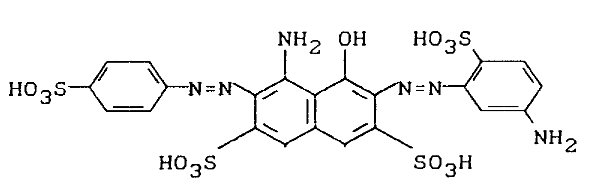

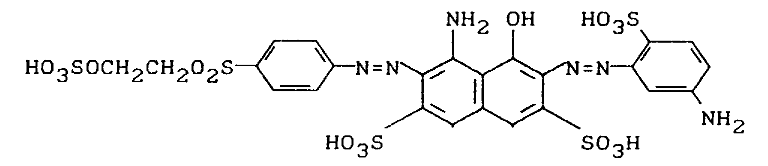

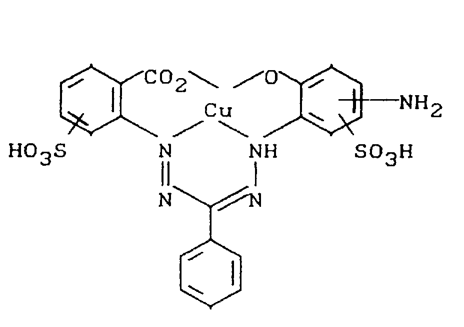

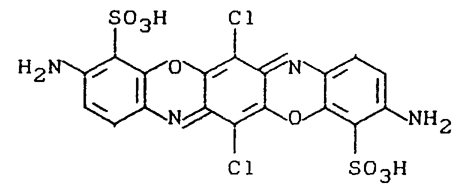

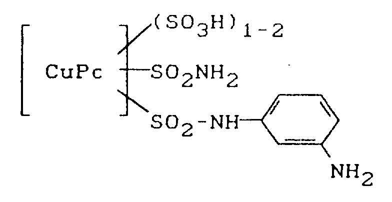

Die Umsetzung von (II) mit (III) erfolgt im allgemeinen im Molverhältnis 0,8:1 bis 1,5:1, vorzugsweise 1:1 bis 1,2:1, insbesondere 1:1 bis 1,08:1.

Die Umsetzung erfolgt dabei in wäßrigem Medium, im allgemeinen in Lösung, kann aber auch in wäßriger Suspension durchgeführt werden. Die Umsetzungstemperaturen liegen bei -5°C bis +20°C, vorzugsweise -2°C bis +10°C.

Vorteilhafterweise arbeitet man unter einer Schutzgasatmosphäre wie Stickstoff oder Argon.

Die Umsetzung erfolgt bevorzugt in Gegenwart ausreichender Mengen Puffersubstanzen wie Alkalifluoriden, -boraten, -acetaten.

Die Puffersubstanzen werden im allgemeinen in einer Menge von 0,5 bis 2, vorzugsweise 0,8 bis 1,2 Mol je Mol Amin eingesetzt.

Dabei wird zunächst ein pH-Bereich von etwa 2,5 bis 6 eingestellt (Austausch des 1. Fluoratoms) und danach durch Zudosieren von Basen wie Alkalihydroxiden, Alkalicarbonaten oder Alkalibicarbonaten (Li, Na, K) ein pH-Bereich von etwa 6-10.

Die Basen werden in Mengen von etwa 0,5 bis 1,5 Mol pro Mol (II) eingesetzt.

Im einzelnen erfolgt die Umsetzung folgendermaßen:

Die Edukte werden gleichzeitig und kontinuierlich in einen ersten Reaktor geleitet, wo eine intensive Durchmischung erfolgt, z.B. mittels einer geeigneten Dispergiereinheit, wie einem Rotor-Stator-Mischkopf, mit ca. 1.000 bis 25.000 Umdrehungen pro Minute oder einer Ultraschallmischkammer oder einer statischen Mischvorrichtung. Die Verweilzeit in diesem ersten Reaktor soll möglichst kurz sein, sie muß jedoch so bemessen sein, daß eine genügende Durchmischung erfolgt. Im allgemeinen beträgt die Verweilzeit im ersten Reaktor maximal 5 Sekunden, vorzugsweise maximal 1 Sekunde.

Während dieser Zeit erfolgt bereits zum Teil eine Reaktion des Cyanurfluorids mit dem Ammoniumsalz des Amins der Formel (II). Es hat sich als günstig erwiesen, insbesondere falls als Amin eine Aminonaphtholsulfonsäure verwendet wird, wenn der Umsatz in dem ersten Reaktor maximal 50 %, vorzugsweise maximal 30 % beträgt. Dies wird durch geringes Volumen des ersten Reaktors bzw. durch kurze Verweilzeit erreicht.

Aus dem ersten Reaktor wird die Reaktionsmischung in einen zweiten Reaktor geleitet und dort die erste Kondensation zu Ende geführt. Als zweiter Reaktor ist generell jeder Reaktor geeignet, in dem nur geringe Rückvermischung aber gute radiale Vermischung eintritt. Vorzugsweise verwendet man einen Rohrreaktor mit gutem Pfropfströmungsprofil und arbeitet im turbulenten Strömungsbereich. Rohrreaktoren mit laminarer Strömung sind ebenfalls verwendbar, wenn sie mit geeigneten statischen Mischelementen zur Verbesserung der radialen Durchmischung ausgerüstet sind. Dieser zweite Reaktor wird gegebenenfalls gekühlt, um die Temperatur im gewünschten Bereich zu halten. Die Verweilzeit der Reaktionsmischung im zweiten Reaktor hängt u.a. von der Art des Amins und der Temperatur ab, im allgemeinen liegt sie etwa zwischen 30 Sekunden und 5 Minuten.

Dabei wird zunächst ein pH-Bereich von etwa 2,5 - 6 durchlaufen und danach durch Zudosieren von Basen, wie z.B. Alkalihydroxid, Alkalicarbonat oder Alkalihydrogencarbonat, ein pH-Bereich von 6 - 10 bis zum vollständigen Austausch des zweiten Halogenatoms eingestellt.

Die Erhöhung des pH-Wertes wird vorzugsweise entweder am Ende des zweiten Reaktors oder aber in einem dritten Reaktor durchgeführt. Selbstverständlich kann die pH-Regulierung auch in einem Rührwerkskessel erfolgen.

Die Umsetzung erfolgt überraschenderweise schnell, in hohen Ausbeuten und führt zu sehr einheitlichen Reaktionsprodukten. Unerwünschte symmetrische Bis-Kondensationsprodukte treten allenfalls in Spuren auf.

Die Nachteile der bekannten zweistufigen Verfahren (vgl. DE-A 2 741 011, 2 903 594, 3 545 460, 2 557 141 und EP-A 172 790; uneinheitliche Reaktionsprodukte, Hydrolyseprodukte) treten hier nicht auf.

Die erhaltenen Reaktionsprodukte bzw. Reaktivfarbstoffe der Formel (I) können isoliert werden, sie werden jedoch im Falle von A = nichtchromophorer Rest vorzugsweise ohne Zwischenisolierung zu Reaktivfarbstoffen weiterverarbeitet.

Diese Weiterverarbeitung - Ankuppeln oder Diazotieren von (I), - kann diskontinuierlich oder kontinuierlich auf bekannte Art und Weise durchgeführt werden.

Gegenstand der Erfindung ist demzufolge auch ein Verfahren zur Herstellung von Reaktivfarbstoffen, bei dem zunächst gemäß Anspruch 1 Verbindungen der Formel I mit A = Rest einer Kupplungskomponente oder Rest einer Diazokomponente hergestellt und diese anschließend ohne Zwischenisolierung kontinuierlich oder diskontinuierlich mit diazotierten Aminen in üblicher Weise gekuppelt bzw. diazotiert und ohne Zwischenisolierung kontinuierlich oder diskontinuierlich mit Kupplungskomponenten gekuppelt werden.

### Beispiel 1

In einen ersten Reaktor (Dispergiereinheit) werden gleichzeitig und kontinuierlich über getrennte Zuleitungen 8,8 ml (0,103 Mol) Trifluor-s-triazin mit einer Temperatur von 20-25°C sowie 200 ml einer wäßrigen Lösung von 0°C, die durch Zugabe von 34,1 g (0,1 Mol) mono-Natriumsalz der 1-Hydroxy-8-aminonaphthalin-3,6-disulfonsäure, 9,0 g (0,103 Mol) Morpholin, 5 g (0,12 Mol) Natriumfluorid sowie 40 g Eis zu 140 ml Wasser bereitet wird, eingespeist.

In dem Reaktor wird durch eine Dispergiervorrichtung während einer Verweilzeit von 0,2-1,0 Sekunden bei hoher Turbulenz eine gleichmäßige, schnelle Durchmischung der Phasen erreicht. Aus dem ersten Reaktor wird die Reaktionsmischung durch einen gekühlten Rohrreaktor mit gutem Pfropf-Strömungsprofil geleitet. Die Verweilzeit im Rohrreaktor beträgt etwa 15-30 Sekunden.

Das Reaktionsgemisch verläßt den Reaktor mit einer Temperatur von ca. 3°C und einem pH-Wert von ca. 4,2. Die erhaltene Reaktionsmischung wird in einem Rührkessel geleitet und dort innerhalb von 10 Minuten durch Zutropfen einer Sodalösung auf pH 7,0 eingestellt und bei diesem Wert gehalten. Danach kristallisiert das Kondensationsprodukt der Formel 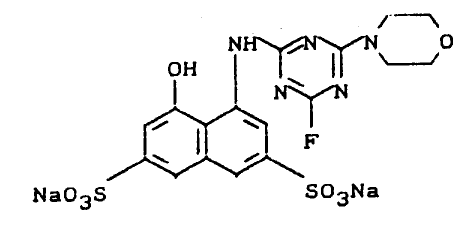 langsam aus. Es kann durch Absaugen isoliert werden. Aufgrund der einheitlich verlaufenden Kondensationsreaktionen kann die Reaktionsmischung aber auch direkt weiter umgesetzt werden; so kann sie z.B. mit diazotierter 2-Aminonaphthalin-1-sulfonsäure zu einem wertvollen roten Monofluortriazin-Reaktivfarbstoff gekuppelt werden.

Ersetzt man in Beispiel 1 Morpholin durch eine äquivalente Menge eines anderen Amins, z.B. Dimethylamin, Diethylamin, Piperidin, Methylamin, Benzylamin oder 2-(Methylamino)ethanol und arbeitet ansonsten wie beschrieben, so führt das Verfahren ebenfalls zu den entsprechend substituierten einheitlichen Dikondensationsprodukten der Formel

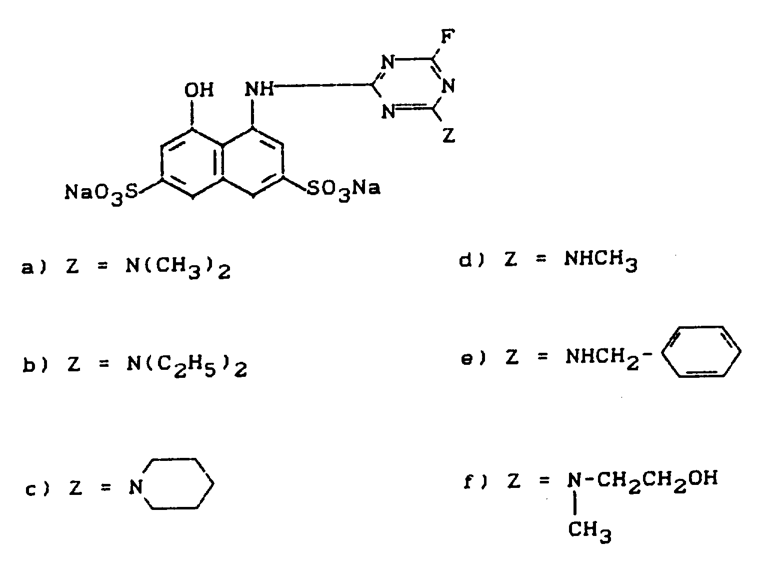

### Beispiel 2

23,9 g (0,10 Mol) 1-Hydroxy-6-aminonaphthalin-3-sulfonsäure werden in 200 ml Wasser angerührt und durch Zugabe von 6,3 g (0,103 Mol) 2-Aminoethanol gelöst. Es wird mit 5 g (0,12 Mol) Natriumfluorid und 50 g Eis versetzt und mit verdünnter Salzsäure auf pH 6,0 gestellt. Die Lösung wird gleichzeitig und kontinuierlich über getrennte Zuleitungen gemeinsam mit 8,8 ml 0,103 Mol) Trifluortriazin in den ersten Reaktor eingespeist. Man verfährt weiter, wie in Beispiel 1 beschrieben, und erhält nach Sodazugabe eine Reaktionsmischung des Dikondensationsproduktes der Formel 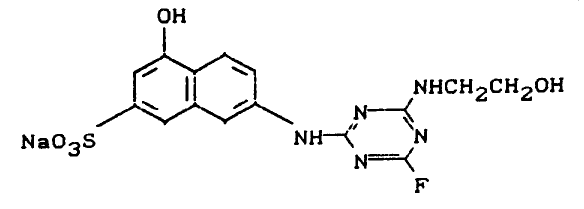 das ohne Zwischenisolierung mit diazotierten Anilinen zu interessanten Azoreaktivfarbstoffen umgesetzt werden kann.

### Beispiel 3

Verwendet man in Beispiel 1 anstelle von 1-Hydroxy-8-aminonaphthalin-3,6-disulfonsäure-mono-Natriumsalz nun 18,8 g (0,1 Mol) 2,4-Diaminobenzolsulfonsäure und verfährt ansonsten ganz analog, so resultiert ein relativ einheitliches Dikondensationsprodukt der Formel 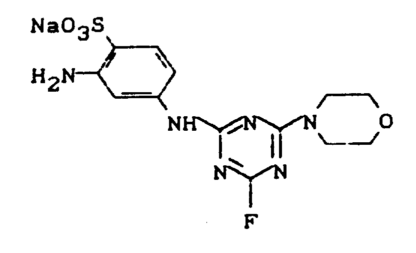 das durch Diazotieren bei 0°C und pH 2,0 bis 2,5 in eine wertvolle Diazoniumsalzkomponente überführt werden kann.

### Beispiel 4

Verwendet man in Beispiel 1 anstelle der farblosen 1-Hydroxy-8-aminonaphthalin-3,6-disulfonsäure (mono-Natriumsalz) nun 59,5 g (0,101 Mol) der Farbbase der Formel 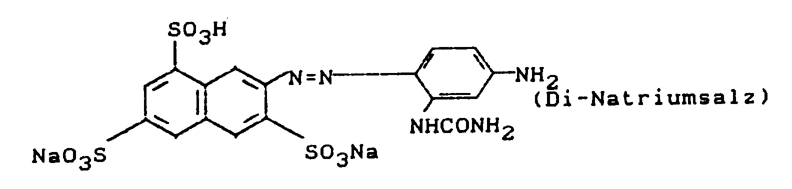 und führt die Kondensationen in Analogie zu Beispiel 1 durch, so resultiert ein Reaktivfarbstoff der Formel 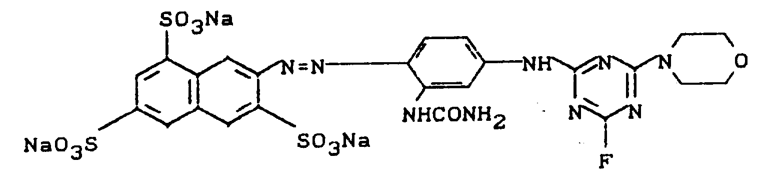 der durch Aussalzen isoliert wird und Baumwolle in klaren hochechten goldgelben Farbtönen färbt.

## Patentansprüche

1. Verfahren zur Herstellung von substituierten 2,4-Diamino-6-fluortriazinen der Formel 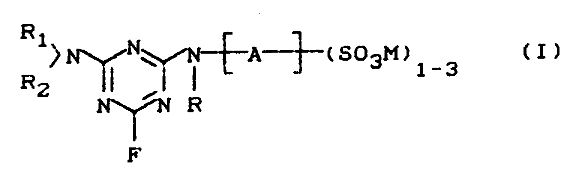 dadurch gekennzeichnet, daß man Ammoniumsalze der Formel 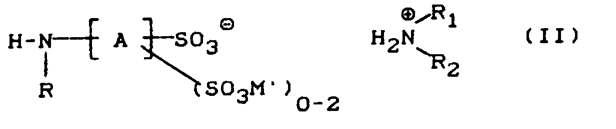 worin
A = aromatisch-carbocyclischer oder aromatischheterocyclischer Rest
M = Alkalimetallkation
R = H oder C₁-C₄-Alkyl
R₁, R₂ = unabhängig voneinander Wasserstoff, aliphatischer oder cycloaliphatischer Rest oder zusammen mit dem N-Atom ein 5- oder 6-gliedriger heterocyclischer Rest und
M' = M oder H bedeuten,
in wäßrigen Medien bei pH-Werten von etwa 2,5-10,0 kontinuierlich mit 2,4,6-Trifluortriazin (III) im Molverhältnis II zu III von 0,8:1 bis 1,5:1 umsetzt, wobei man das Triazin III und eine wässrige Lösung des Ammoniumsalzes (II) gleichzeitig und kontinuierlich in einen ersten Reaktor leitet, dort eine intensive Durchmischung vornimmt, und die Reaktionsmischung anschließend in einen zweiten Reaktor leitet, in dem nur geringe Rückmischung aber gute radiale Vermischung eintritt, und dort die Erstkondensation bei pH 2,5 bis 6 zu Ende führt und anschließend im 2. Reaktor kontinuierlich oder in einem dritten Reaktor kontinuierlich oder diskontinuierlich bei pH 6 bis 10, durch Zudosieren von Basen, den Austausch des 2. Fluoratoms durchführt.

2. Verfahren nach Anspruch 1, dadurch gekennzeichnet, daß in Gegenwart von Puffersubstanzen, in Mengen von 0,5 bis 2 Mol pro Mol (II) kondensiert wird.

3. Verfahren nach wenigstens einem der vorhergehenden Ansprüche, dadurch gekennzeichnet, daß zunächst in Gegenwart von Puffersubstanzen und Abwesenheit starker Basen wie Alkalien im pH-Bereich 2,5 bis 6 kondensiert wird und danach durch Zusatz starker Basen im pH-Bereich 6 bis 10 die Kondensation zu Ende geführt wird.

4. Verfahren nach Anspruch 1, dadurch gekennzeichnet, daß als Base Alkalicarbonat oder Alkalihydrogencarbonat verwendet wird.

5. Verfahren nach Anspruch 1, dadurch gekennzeichnet, daß die Base in Mengen von 0,5 bis 1,5 Mol pro Mol der Verbindung der Formel II eingesetzt wird.

6. Verfahren nach wenigstens einem der vorhergehenden Ansprüche, dadurch gekennzeichnet, daß die Umsetzung bei -5°C bis +20°C vorgenommen wird.

7. Verfahren nach wenigstens einem der vorhergehenden Ansprüche, dadurch gekennzeichnet, daß R¹ und R unabhänig voneinander CH₃, C₂H₅, C₃H₇, C₄H₉ (verzweigt bzw. geradkettig), CH₂CH₂OH, CH₂CH₂CN, CH₂CO₂H, CH₂CH₂CO₂H, CH₂CH₂SO₃H, CH₂SO₃H, CH₂CH₂OSO₃H, CH₂CH₂CH₂OH, CH₂CH₂CH₂OSO₃H, CH₂-CH(OH)-CH₃, CH₂CH₂NHCOCH₃, CH₂CH₂OCH₃, CH₂CH₂OCH₂CH₃, CH₂CH₂CH₂OC₂H₅, CH₂CH₂N(CH₃)₂, CH₂CH₂CH₂N(CH₃)₂, Cyclohexyl, Benzyl, Sulfobenzyl, CH₂CH₂-C₆H₅ sowie 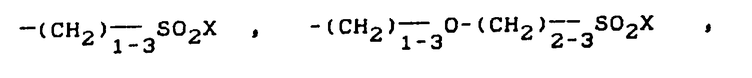 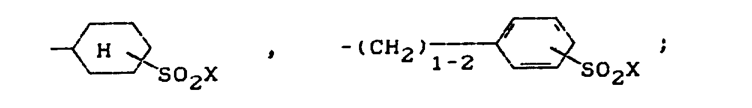 oder zusammen mit dem N-Atom 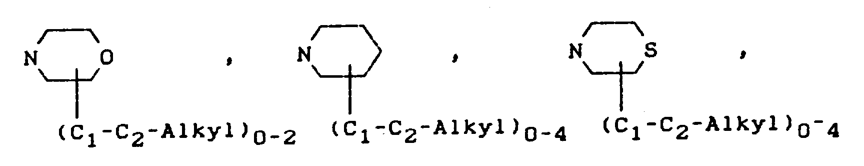 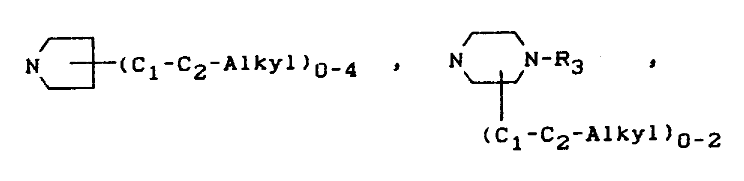 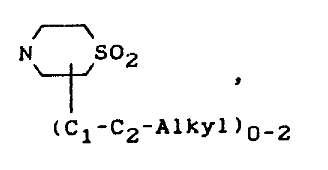 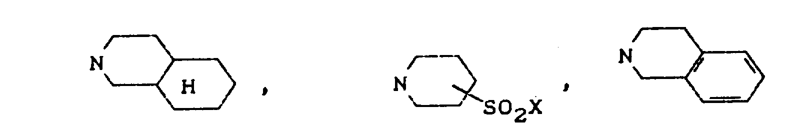 bedeuten, wobei
X = CH=CH₂, C₂H₄OSO₃H, C₂H₄Cl, C₂H₄SSO₃H, C₂H₄OCOCH₃
R₃ = H, gegebenenfalls substituiertes C₁-C₄-Alkyl.

8. Verfahren nach wenigstens einem der vorhergehenden Ansprüche, dadurch gekennzeichnet, daß A ein Benzol- oder ein Naphthalinrest ist.

9. Verfahren nach wenigstens einem der vorhergehenden Ansprüche, dadurch gekennzeichnet, daß A in Form der freien Säure eine der folgenden Bedeutungen aufweist

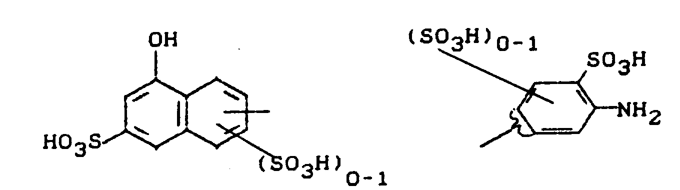

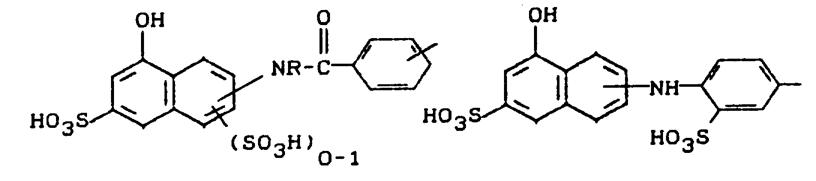

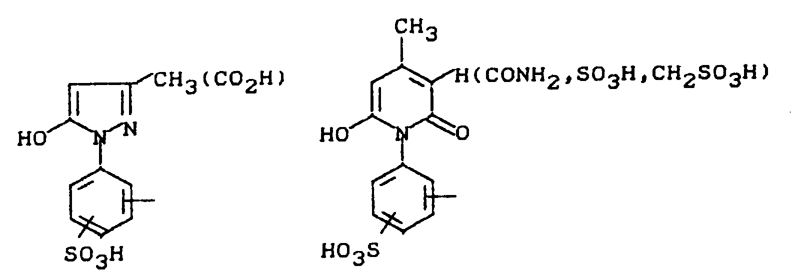

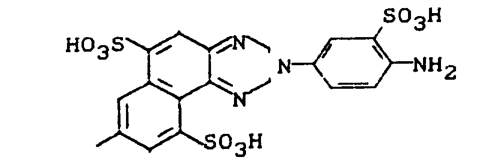

## Claims

1. Process for preparing substituted 2,4-diamino-6-fluorotriazines of the formula 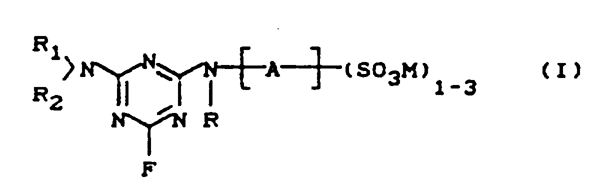 characterized in that ammonium salts of the formula 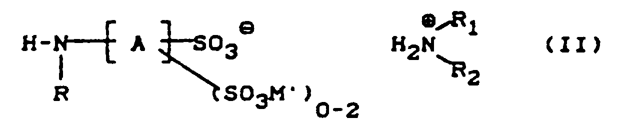 in which
A denotes an aromatic carbocyclic or aromatic heterocyclic radical,
M denotes an alkali metal cation,
R denotes H or C₁-C₄-alkyl,
R₁, R₂ denote, independently of one another, hydrogen, an aliphatic or cycloaliphatic radical or together with the N atom denote a 5- or 6-membered heterocyclic radical and
M' denotes M or H,
are reacted continuously with 2,4,6-trifluorotriazine (III) in a II/III molar ratio of 0.8:1 to 1.5:1 in aqueous media at pH values of about 2.5-10.0, triazine III and an aqueous solution of the ammonium salt (III) being introduced simultaneously and continuously into a first reactor, the reaction mixture being vigorously mixed there and subsequently being passed into a second reactor in which only slight backmixing but efficient radial mixing takes place, the first condensation being brought to completion there at a pH of 2.5 to 6, and exchange of the second fluorine atom then being effected continously in the second reactor or continously or batchwise in a third reactor at a pH of 6 to 10 by metered addition of bases.

2. Process according to Claim 1, characterized in that the condensation is carried out in the presence of buffer substances in amounts of 0.5 to 2 mol per mole of (II).

3. Process according to at least one of the preceding claims, characterized in that first the condensation is carried out in the pH range of 2.5 to 6 in the presence of buffer substances and in the absence of strong bases such as alkalis and the condensation is then brought to completion in the pH range of 6 to 10 by addition of strong bases.

4. Process according to Claim 1, characterized in that the base used is alkali metal carbonate or alkali metal bicarbonate.

5. Process according to Claim 1, characterized in that the base is used in amounts of 0.5 to 1.5 mol per mole of the compound of the formula II.

6. Process according to at least one of the preceding claims, characterized in that the reaction is carried out at -5°C to +20°C.

7. Process according to at least one of the preceding claims, characterized in that R¹ and R denote, independently of one another, CH₃, C₂H₅, C₃H₇, C₄H₉ (branched or straight-chain), CH₂CH₂OH, CH₂CH₂CN, CH₂CO₂H, CH₂CH₂CO₂H, CH₂CH₂SO₃H, CH₂SO₃H, CH₂CH₂OSO₃H, CH₂CH₂CH₂OH, CH₂CH₂CH₂OSO₃H, CH₂-CH(OH)-CH₃, CH₂CH₂NHCOCH₃, CH₂CH₂OCH₃, CH₂CH₂OCH₂CH₃, CH₂CH₂CH₂OC₂H₅, CH₂CH₂N(CH₃)₂, CH₂CH₂CH₂N(CH₃)₂, cyclohexyl, benzyl, sulphobenzyl, CH₂CH₂-C₆H₅ and 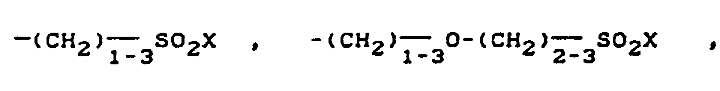 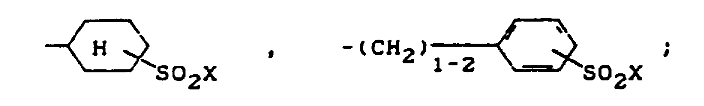 or together with the N atom 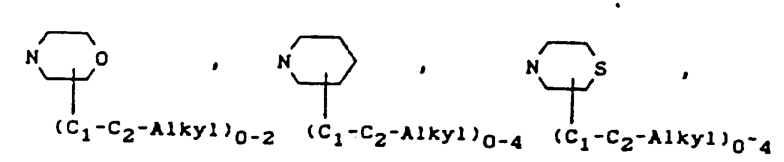 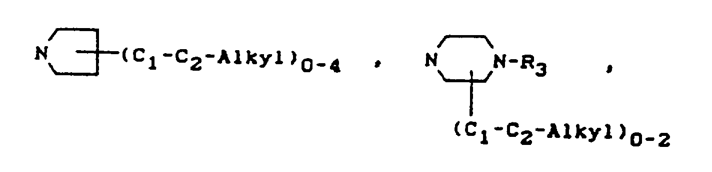 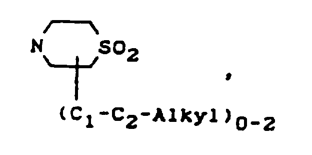 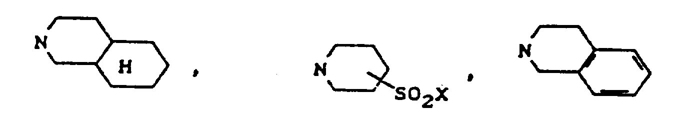 where
X denotes CH=CH₂, C₂H₄OSO₃H, C₂H₄Cl, C₂H₄SSO₃H, C₂H₄OCOCH₃,
R₃ denotes H or substituted or unsubstituted C₁-C₄-alkyl.

8. Process according to at least one of the preceding claims, characterized in that A denotes a benzene or a naphthalene radical.

9. Process according to at least one of the preceding claims, characterized in that A in the form of the free acid has one of the following meanings

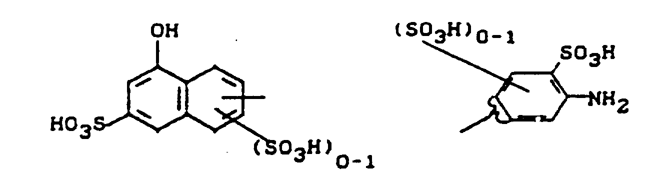

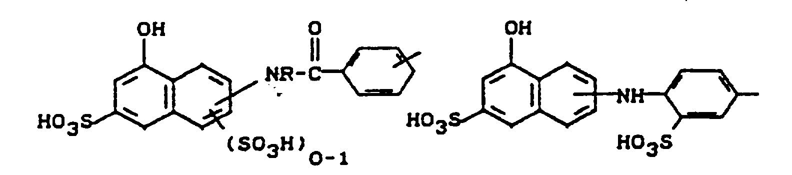

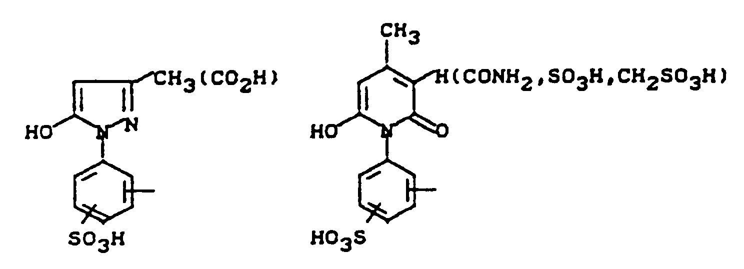

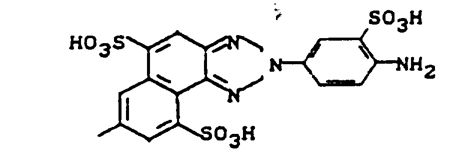

## Revendications

1. Procédé pour la préparation des 2,4-diamino-6-fluorotriazines substituées de formule 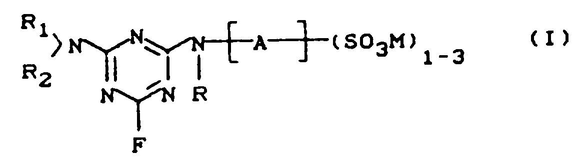 caractérisé en ce que l'on fait réagir des sels d'ammonium de formule 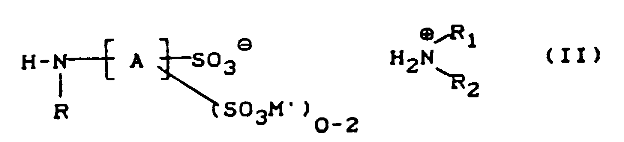 dans lesquelles
A représente un radical aromatique homogène ou hétérogène,
M représente un cation de métal alcalin,
R représenté H ou un groupe alkyle en C₁-C₄,
R₁ et R₂ représentent chacun, indépendamment l'un de l'autre, l'hydrogène, un radical aliphatique ou cycloaliphatique, ou bien forment ensemble et avec l'atome d'azote un radical hétérocyclique à cinq ou six chaînons et
M' a les mêmes significations que M ou représente H,
en milieu aqueux, à des pH d'environ 2,5 à 10,0, en continu, avec la 2,4,6-trifluorotriazine III dans un rapport molaire II/III de 0,8 : 1 à 1,5 : 1, en envoyant la triazine III et une solution aqueuse du sel d'ammonium II simultanément et en continu dans un premier réacteur où l'on réalise un mélange intensif, puis en envoyant le mélange de réaction dans un deuxième réacteur dans lequel il n'y a qu'un faible mélange en retour, mais un bon mélange radial, et dans lequel on achève la première condensation à un pH de 2,5 à 6 puis, dans le deuxième réacteur, en continu, ou bien dans un troisième réacteur, en continu ou en discontinu, en procédant à l'échange du deuxième atome de fluor à un pH de 6 à 10, par addition d'une base.

2. Procédé selon la revendication 1, caractérisé en ce que l'on condense en présence de substances tampons en quantités de 0,5 à 2 mol par mole de II.

3. Procédé selon au moins une des revendications qui précèdent, caractérisé en ce que l'on condense d'abord en présence de substances tampons et en l'absence de bases fortes telles que des alcalis dans l'intervalle de pH de 2,5 à 6, puis, par addition de bases fortes, on achève la condensation dans l'intervalle de pH de 6 à 10.

4. Procédé selon la revendication 1, caractérisé en ce que les bases utilisées sont des carbonates ou bicarbonates alcalins.

5. Procédé selon la revendication 1, caractérisé en ce que les bases sont mises en oeuvre en quantité de 0,5 à 1,5 mol par mole du composé de formule II.

6. Procédé selon au moins une des revendications qui précèdent, caractérisé en ce que la réaction est effectuée à des températures allant de -5 à +20°C.

7. Procédé selon au moins une des revendications qui précèdent, caractérisé en ce que R₁ et R₂ représentent chacun, indépendamment l'un de l'autre, CH₃, C₂H₅, C₃H₇, C₄H₉ (à chaîne droite ou ramifiée), CH₂CH₂OH, CH₂CH₂CN, CH₂CO₂H, CH₂CH₂CO₂H, CH₂CH₂SO₃H, CH₂SO₃H, CH₂CH₂OSO₃H, CH₂CH₂CH₂OH, CH₂CH₂CH₂OSO₃H, CH₂-CH(OH)-CH₃, CH₂CH₂NHCOCH₃, CH₂CH₂OCH₃, CH₂CH₂OCH₂CH₃, CH₂CH₂CH₂OC₂H₅, CH₂CH₂N(CH₃)₂, CH₂CH₂CH₂N(CH₃)₂, cyclohexyle, benzyle, sulfobenzyle, CH₂CH₂-C₆H₅ et 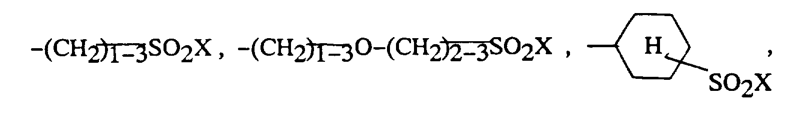 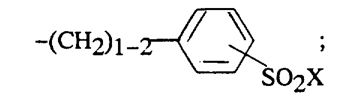 ou bien forment ensemble et avec l'atome d'azote 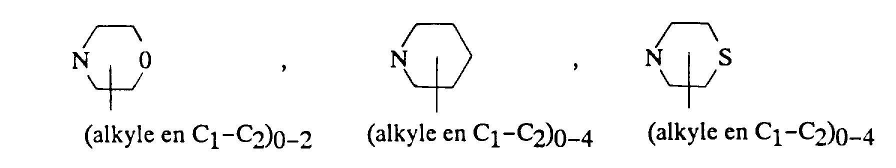 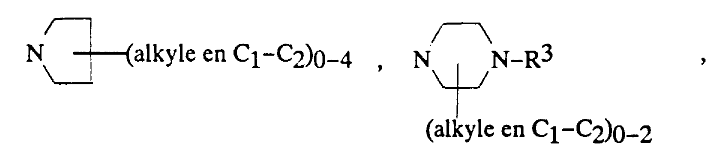 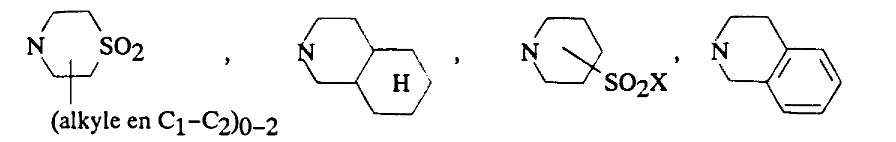 dans lesquels
X représente CH=CH₂, C₂H₄OSO₃H, C₂H₄Cl, C₂H₄SSO₃H, C₂H₄OCOCH₃ et
R₃ représente H, un groupe alkyle en C₂-C₄ éventuellement substitué.

8. Procédé selon au moins une des revendications qui précèdent, caractérisé en ce que A est un radical benzénique ou naphtalénique.

9. Procédé selon au moins une des revendications qui précèdent, caractérisé en ce que A, à l'état d'acide libre, consiste en l'un des radicaux suivants :
